(19) Europäisches Patentamt / European Patent Office / Office européen des brevets

(11) EP 2 301 522 B1

# (12) EUROPEAN PATENT SPECIFICATION

(45) Date of publication and mention of the grant of the patent:
**28.02.2018 Bulletin 2018/09**

(21) Application number: **10776516.6**

(22) Date of filing: **03.06.2010**

(51) Int Cl.:
***A61K 8/89*** (2006.01) ***A61K 8/19*** (2006.01)
***A61K 8/29*** (2006.01) ***A61K 8/34*** (2006.01)
***A61K 8/06*** (2006.01) ***A61Q 1/02*** (2006.01)

(86) International application number:
**PCT/JP2010/059425**

(87) International publication number:
**WO 2011/001781 (06.01.2011 Gazette 2011/01)**

## (54) WATER-IN-OIL EMULSION-TYPE COSMETIC PREPARATION

WASSER-IN-ÖL-EMULSIONS-KOSMETIKPRÄPARAT

PRÉPARATION COSMÉTIQUE DU TYPE ÉMULSION EAU DANS L'HUILE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **12.02.2010 JP 2010028496**

(43) Date of publication of application:
**30.03.2011 Bulletin 2011/13**

(73) Proprietor: **Shiseido Company, Ltd.**
**Chuo-ku,**
**Tokyo 104-0061 (JP)**

(72) Inventors:
- **MUNE, Yoriko**
  **Kanagawa 224-8558 (JP)**
- **HATA, Hideo**
  **Kanagawa 224-8558 (JP)**
- **SATO, Yukiko**
  **Kanagawa 224-8558 (JP)**
- **HASEGAWA, Katsuyuki**
  **Kanagawa 224-8558 (JP)**

(74) Representative: **Henkel, Breuer & Partner**
**Patentanwälte**
**Maximiliansplatz 21**
**80333 München (DE)**

(56) References cited:
**EP-A1- 0 331 833** **EP-A1- 1 374 835**
**EP-A2- 1 064 909** **WO-A1-2007/091490**
**JP-A- S62 216 635** **JP-A- S63 151 351**
**JP-A- 2000 256 127** **JP-A- 2000 351 712**
**JP-A- 2001 226 222** **JP-A- 2001 278 738**
**JP-A- 2002 193 741** **JP-A- 2007 161 650**
**JP-A- 2007 269 689** **JP-A- 2007 269 690**
**JP-A- 2008 247 816** **JP-A- 2009 084 171**

Remarks:
The file contains technical information submitted after the application was filed and not included in this specification

EP 2 301 522 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### RELATED APPLICATIONS

This application claims the priority of Japanese Patent Application No. 2010-28496 filed on February 12, 2010.

### FIELD OF THE INVENTION

The present invention relates to W/O emulsion cosmetics, and in particular, relates to the improvement of the feeling in use and emulsion stability thereof.

### BACKGROUND OF THE INVENTION

W/O emulsions are long-lasting compared with O/W emulsions; therefore, they are widely used in cosmetics, and in particular, in makeup cosmetics such as foundation and sunscreen cosmetics. However, further improvement of the feeling in use is desired.

It is reported in Patent Literature 1 (Japanese Examined Patent Publication No. S63-250311 A, Kao Corporation) that W/O type cosmetics excellent in the cooling sensation and emulsion stability can be obtained by using a specific dimethylpolysiloxane polyoxyalkylene copolymer as the emulsifying agent and blending with a silicone oil and ethanol.

However, even such W/O type cosmetics were not satisfactory enough in the feeling in use and stability.

Patent Literature 1: Japanese Examined Patent Application Publication No. S63-250311 A

### SUMMARY OF THE INVENTION

### PROBLEM TO BE SOLVED BY THE INVENTION

The present invention was made in view of the above-described background art, and an object thereof is to provide a W/O emulsion cosmetic excellent in the feeling in use and emulsion stability.

### MEANS TO SOLVE THE PROBLEM

The present inventors have diligently studied to solve the above-described problems. As a result, the present inventors have found that a W/O emulsion cosmetic excellent in the feeling in use and with good emulsion stability can be obtained by blending a specific modified silicone in the W/O emulsion cosmetic containing powder and volatile components such as a volatile oil and ethanol. In addition, the present inventors have found that the W/O emulsion cosmetic displays a characteristic friction behavior. Furthermore, the present inventors have also found that the dispersion stability of powder, viscosity stability of compositions, or the moisturizing property can be improved by further blending the specific component therein without detriment to the feeling in use and emulsion stability, thus leading to completion of the present invention.

That is, the present invention provides a W/O emulsion cosmetic as defined in the claims.

### EFFECT OF THE INVENTION

According to the present invention, a W/O emulsion cosmetic that shows a characteristic friction behavior and are excellent in the feeling in use and emulsion stability can be obtained by using (A) a polyoxyalkylene/alkyl-comodified silicone, (B) a polyoxyalkylene-modified silicone, (C) powder, (D) a volatile oil, and (E) ethanol in a specific ratio. In addition, the dispersion stability of powder and viscosity stability of the composition can be improved, by further blending an organically modified clay mineral and diglyceryl diisostearate therein, without detriment to the feeling in use and emulsion stability. Furthermore, if glycerin and one or more nonvolatile oils selected from the group consisting of diethylhexyl succinate, methylphenylpolysiloxane, and dimethylpolysiloxane are blended therein, the moisturizing property can be improved without detriment to the feeling in use and emulsion stability.

### BRIEF DESCRIPTION OF THE DRAWINGS

Fig. 1 shows the comparison of kinetic friction force curves for the W/O emulsion cosmetics in which (A) a polyoxyalkylene/alkyl-comodified silicone was used (Sample 1-1 and Sample 1-2) and the W/O emulsion cosmetics in which

(B) a polyoxyalkylene-modified silicone was used (Sample 1-3 and Sample 1-4).
Fig. 2 shows the change in the kinetic friction force curve when the type of the volatile oil was changed.
Fig. 3 illustrates (a) kinetic friction force curve pattern A, which is characteristic to the present invention, and (b) kinetic friction force curve pattern B, which is a comparative example.

## BEST MODE FOR CARRYING OUT THE INVENTION

### (A) Polyoxyalkylene/alkyl-comodified silicone

The polyoxyalkylene/alkyl-comodified silicone used in the present invention has a linear or branched organopolysiloxane chain as the main backbone and has a polyoxyalkylene group and an alkyl group having 4 or more carbon atoms on the side chains. The polyoxyalkylene/alkyl-comodified silicone is represented by the following formula (I).

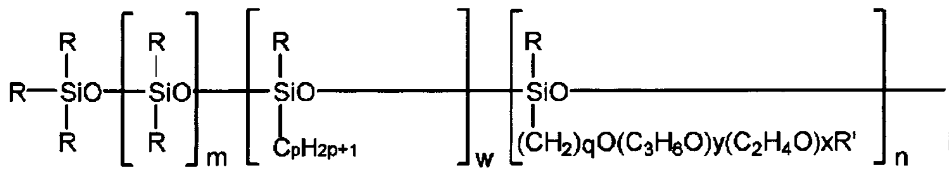
· · · (I)

In the formula (I),
R is an alkyl group of 1 to 3 carbon atoms or a phenyl group (preferably a methyl group);
R' is a hydrogen atom or an alkyl group of 1 to 12 carbon atoms (preferably a hydrogen atom);
p is 6 to 30 (preferably 10 to 18, and more preferably 12 to 16);
q is 1 to 50 (preferably 3);
m is 1 to 100;
n, w, and x are 1 to 50, respectively; and
y is 1 to 50.

As one preferable example of polyoxyalkylene/alkyl-comodified silicone represented by the formula (I), ABIL EM90 (manufactured by Goldschmidt, Germany) can be listed.

In the above-described formula (I), the organopolysiloxane main backbone may have another organopolysiloxane chain as a side chain. As one preferable example of such a polyoxyalkylene/alkyl-comodified silicone, KF-6038 (lauryl PEG-9 polydimethylsiloxyethyl dimethicone, manufactured by Shin-Etsu Chemical Co., Ltd.) can be listed.

The polyoxyalkylene/alkyl-comodified silicone, by the interaction with powder, allows the W/O emulsion cosmetic to easily adapt to the skin with a swift finish and generate an excellent feeling in use with the right fit. Such a feeling in use unique to the present invention is related, as described below, to the friction behavior of the W/O emulsion composition. The amount of the polyoxyalkylene/alkyl-comodified silicone is 0.1 to 5 mass % in the W/O emulsion cosmetics, preferably 0.3 to 3 mass %, and more preferably 1 to 2 mass %. If the amount of the polyoxyalkylene/alkyl-comodified silicone is too small, the effect is not satisfactory. On the other hand, even when the excess amount is blended, the corresponding improvement in the effect cannot be achieved.

### (B) Polyoxyalkylene-modified silicone

The polyoxyalkylene-modified silicone used in the present invention has a linear or branched organopolysiloxane chain as the main backbone and has a polyoxyalkylene group on the side chain. The polyoxyalkylene-modified silicone is represented by the following formula (II).

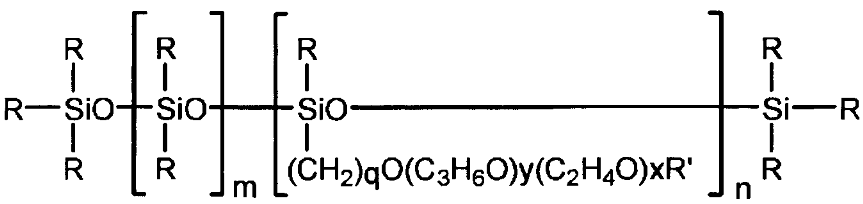
· · · (II)

In the formula (II),
R is an alkyl group of 1 to 3 carbon atoms or a phenyl group (preferably a methyl group);
R' is a hydrogen atom or an alkyl group of 1 to 12 carbon atoms (preferably a hydrogen atom or a methyl group);

q is 1 to 50 (preferably 3);
m is 1 to 100;
n and x are 1 to 50, respectively; and
y is 1 to 50.

As one preferable example of polyoxyalkylene-modified silicone of the formula (II), KF-6017 (PEG-10 dimethicone, manufactured by Shin-Etsu Chemical Co., Ltd.) can be listed.

In the above-described formula (II), the organopolysiloxane main backbone may have another organopolysiloxane chain as a side chain. As one preferable example of such a polyoxyalkylene-modified silicone, KF-6028 (PEG-9 polydimethylsiloxyethyl dimethicone, manufactured by Shin-Etsu Chemical Co., Ltd.) can be listed.

The polyoxyalkylene-modified silicone contributes to emulsion stability. The blending quantity in the W/O emulsion cosmetics is normally 0.1 to 5 mass %, and typically 0.3 to 3 mass %. The blending quantity of the polyoxyalkylene-modified silicone with respect to the polyoxyalkylene/alkyl-comodified silicone is 0.2 to 3 times in mass, and preferably 0.5 to 2 times in mass. If the blending quantity of the polyoxyalkylene-modified silicone is too small, the emulsion stabilizing effect is not satisfactory. On the other hand, if an excess amount is blended, the excellent feeling in use of the present invention is undermined.

**(C) Powder**

The powder used in the present invention is not limited in particular so far as it is normally used in cosmetics, and one or more can be used. Examples thereof include inorganic white pigments such as talc, kaolin, sericite, muscovite and titanium oxide; inorganic red pigments such as iron oxide (bengala) and iron titanate; inorganic yellow pigments such as yellow iron oxide and ocher; inorganic violet pigments such as manganese violet and cobalt violet; inorganic green pigments such as chromium oxide, chromium hydroxide, and cobalt titanate; inorganic blue pigments such as ultramarine and Prussian blue; pearly pigments such as titanium oxide-coated mica, titanium oxide-coated bismuth oxychloride, bismuth oxychloride, titanium oxide-coated talc, fish scale flake, and colored titanium oxide-coated mica; metal powder pigments such as aluminum powder and copper powder; inorganic powders such as synthetic mica, phlogopite, lepidolite, biotite, lithia mica, vermiculite, magnesium carbonate, calcium carbonate, diatomaceous earth, magnesium silicate, calcium silicate, aluminum silicate, barium silicate, strontium silicate, metal tungstates, $\alpha$-iron oxide, hydrated iron oxide, silica, and hydroxyapatite; and organic powders such as nylon powder, polyethylene powder, benzoguanamine powder, microcrystalline cellulose, and silicone powder. In addition, the examples include UV protection powders such as fine-particle titanium oxide and fine-particle zinc oxide; and composite powders in which an organic powder is coated with an inorganic powder.

In the present invention, it is preferable to use powder hydrophobized by a publicly known method, such as methods with metallic soaps, silicones, fatty acid esters, etc..

The effect of the present invention becomes prominent when the blending quantity of powder in the W/O emulsion cosmetic is 5 mass % or higher and typically 10 mass % or higher, and it becomes more prominent when 20 mass % or higher. However, if the amount of powder is too much, there are cases where the powder cannot be sufficiently dispersed to take place the aggregation or lower the feeling in use. Therefore, the blending quantity of powder in the W/O emulsion cosmetic is 50 mass % or lower, preferably 40 mass % or lower, and more preferably 30 mass % or lower.

**(D) Volatile oil**

Examples of the volatile oil used in the present invention include isoparaffinic hydrocarbon oils and silicone oils, having low-boiling point (260 °C or lower at ordinary pressure), and one or more can be used.

An example of a low-boiling isoparaffinic hydrocarbon oil includes isododecane.

Examples of low-boiling silicone oils include cyclic dimethylpolysiloxanes having 4 to 6 silicon atoms and chain dimethylpolysiloxanes having 2 to 5 silicon atoms.

The volatile oil contributes to the feeling in use, and in particular, to the feeling in use at the start of application on the skin. The blending quantity of the volatile oil in the W/O emulsion cosmetic is 10 to 50 mass %, preferably 20 to 40 mass %, and more preferably 25 to 35 mass %. If the blending quantity is too small, the effect may not be satisfactorily achieved. Even when the excess amount is blended, the effect corresponding to the increase cannot be achieved; on the contrary, the stability may be affected.

**(E) Ethanol**

Ethanol contributes to the feeling in use, and in particular, to the feeling in use at the start of application on the skin and at the termination of application. The blending quantity of ethanol in the W/O emulsion cosmetic is 1 to 20 mass %, preferably 2 to 10 mass %, and more preferably 3 to 8 mass %. If the blending quantity is too small, the effect cannot

be satisfactorily achieved. If the blending quantity is too much, the stability may worsen.

**(F) Water**

In the present invention, the blending quantity of water is not limited in particular, and it can be set in the range in which the production of a W/O emulsion cosmetic is possible. In the W/O emulsion cosmetic, the blending quantity is normally 10 to 50 mass %, preferably 20 to 40 mass %, and more preferably 25 to 35 mass %.

The W/O emulsion cosmetics of the present invention contain the above-described components (A) to (F) as essential components. However, in accordance with the purpose, other components can be blended so far as the effect of the present invention is not undermined.

**(G) Sodium glutamate**

In the present invention, the powder dispersion stability and viscosity stability can be improved by further blending sodium glutamate without detriment to the excellent feeling in use and the emulsion stability due to the above-described essential components. The blending quantity of sodium glutamate in the W/O emulsion cosmetic is preferably 0.1 to 5 mass %, more preferably 0.2 to 3 mass %, and most preferably 0.5 to 2 mass %.

**(H) Organically modified clay mineral**

In the present invention, the powder dispersion stability and viscosity stability are improved by further blending an organically modified clay mineral. The organically modified clay mineral is a clay mineral in which exchangeable cations present between the crystalline layers of a water-swellable clay mineral, namely hectorite, are replaced with an organic polar compound or an organic cation (for example, a quaternary ammonium cationic surfactant). Specific examples thereof include dimethyldistearylammonium hectorite (= Quaternium-18 hectorite) and benzyldimethylstearylammonium hectorite.

Organically modified hectorites are on the market, for example, as "Bentone 38" (= Quaternium-18 hectorite) and "Bentone 27" (= benzyldimethylstearylammonium hectorite) (all of them are manufactured by Rheox, Inc.). One or more organically modified hectorite can be used.

In the present invention, it is preferable to use an organically modified hectorite of plate-like particles, with the average thickness of 0.1 μm or less and the average length of 0.5 to 50 μm, from the view point of the effect. The above-described commercially available organically modified hectorites are normally aggregates with the average thickness of 2 μm or higher. Such an organically modified hectorite is subjected to in-oil flaking treatment with a mechanical shearing force and/or an impact force by a wet bead mill etc., to obtain a fine dispersion, in which the organically modified hectorite is dispersed as plate-like particles with the average thickness of 0.1 μm or less and the average length of 0.5 to 50 μm is dispersed (refer to Japanese Unexamined Patent Publication No. 2009-40720). In the present invention, an organically modified hectorite in such a finely dispersed state in oil is preferably used. The oil that forms the fine dispersion is not limited in particular, and the above-described volatile oil is one of the preferable oils for the formation of the fine dispersion.

If the amount of the organically modified hectorite is too small, the satisfactory effect cannot be obtained. If the amount is too large, the viscosity becomes excessively high. Therefore, the amount in the W/O emulsion cosmetic is 0.05 to 1 mass %, and preferably 0.1 to 0.5 mass %. In addition, the organically modified hectorite is used in combination with the below-described diglyceryl diisostearate.

**(I) Diglyceryl diisostearate**

Since an organically modified hectorite is blended, in the present invention, the dispersion stability and viscosity stability can be improved as described above. However, when the application is stopped at the finish, sudden heaviness may be caused and the feeling in use may be lowered.

Diglyceryl diisostearate suppresses the lowering in the feeling in use without a detrimental effect to the dispersion stability and viscosity stability due to the organically modified hectorite. The blending quantity of diglyceryl diisostearate is 1 to 10 times in mass with respect to the blending quantity of the organically modified hectorite.

**(J) Nonvolatile oil**

In the present invention, the moisturizing effect due to glycerin can be improved by blending at least one or more nonvolatile oils selected from the group consisting of diethylhexyl succinate, methylphenylpolysiloxane, and dimethylpolysiloxane. These specific nonvolatile oils do not undermine the excellent feeling in use due to the above-described

essential components.

The total amount of these specific nonvolatile oils in the W/O emulsion cosmetic is preferably 0.1 to 10 mass %, more preferably 2 to 8 mass %, and most preferably 3 to 5 mass %. If the blending quantity is too small, the moisturizing effect is not satisfactorily achieved. Even when the excess amount is blended, the corresponding improvement in the effect cannot be achieved.

So far as the effect of the present invention is not undermined, a nonvolatile oil other than the above-described diethylhexyl succinate, methylphenylpolysiloxane, and dimethylpolysiloxane can be blended. Examples thereof include hydrocarbons, ester oils, vegetable oils and fats, animal oils and fats, higher alcohols, and higher fatty acids. However, if the blending quantity is too large, the excellent feeling in use due to the above-described essential components may be undermined. Therefore, the blending quantity of the nonvolatile oil other than the above-described diethylhexyl succinate, methylphenylpolysiloxane, and dimethylpolysiloxane in the W/O emulsion cosmetic is preferably 8 mass % or lower, more preferably 5 mass % or lower, and most preferably 3 mass % or lower.

Examples of hydrocarbons include liquid paraffins, paraffins, squalane, squalene, ozokerite, pristane, ceresin, petrolatum, and microcrystalline wax.

Examples of ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexanoate, trimethylolpropane triisostearate, glyceryl trioctanoate, glyceryl triisopalmitate, trimethylolpropane triisostearate, cetyl 2-ethylhexanoate, pentaerythritol tetra-2-ethylhexanoate, glyceryl tri-2-ethylhexanoate, 2-ethylhexyl palmitate, glyceryl trimyristate, glyceryl tri-2-heptylundecanoate, castor oil fatty acid methyl ester, oleyl oleate, acetoglyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, di-2-hexyldecyl adipate, diisopropyl sebacate, and triethyl citrate.

Examples of vegetable oils and fats include avocado oil, camellia oil, macadamia nut oil, corn oil, olive oil, canola oil, sesame oil, castor oil, peanut oil, almond oil, soybean oil, tea seed oil, jojoba oil, and germ oil.

Examples of animal oils and fats include turtle oil, egg oil, and mink oil.

Examples of higher alcohols include oleyl alcohol, isostearyl alcohol, octyldodecanol, decyltetradecanol, jojoba alcohol, cetyl alcohol, and myristyl alcohol. Examples of higher fatty acids include oleic acid, isostearic acid, linoleic acid, linolenic acid, eicosapentaenoic acid, docosahexaenoic acid, palmitic acid, and stearic acid.

As the oil component, the so-called "hydrating oil", which has excellent solubility in water and can absorb (retain) a large amount of water, may be blended. The hydrating oil is an oil having a property of water retention. Especially, the oil having a water-retaining power of 100% or higher, namely, the oil that can retain water of the self weight or higher, is preferable.

Examples of hydrating oils include dipentaerythritol hexaoxystearate, pentaerythritol tetra(behenate/benzoate/ethylhexanoate), phytosteryl macadamia nut oil fatty acid ester, di(phytosteryl, 2-octyldodecyl) N-lauroyl-L-glutamate, glyceryl triisostearate, and macadamia nut oil polyglyceryl-6 esters behenate.

As the oil component, a POE-POP copolymer dialkyl ether may also be blended. The alkyl groups of the POE-POP copolymer dialkyl ether can be the same or different, and they are alkyl groups having 1 to 4 carbon atoms, preferably a methyl group or an ethyl group, and more preferably a methyl group. It is preferable that the amount of POE groups is 20 to 80 weight % with respect to the sum of POE groups and POP groups. The POE-POP can be either random-type or block-type, and preferably random-type. Such POE-POP copolymer dialkyl ethers are described, for example, in Japanese Unexamined Patent Publication No. 2004-83541 and Japanese Unexamined Patent Publication No. 2006-265135.

Examples thereof include POE(9)POP(2) dimethyl ether, POE(7)POP(12) dimethyl ether, POE(14)POP(7) dimethyl ether, POE(17)POP(4) dimethyl ether, POE(10)POP(10) dimethyl ether, POE(6)POP(14) dimethyl ether, POE(15)POP(5) dimethyl ether, POE(25)POP(25) dimethyl ether, POE(27)POP(14) dimethyl ether, POE(55)POP(28) dimethyl ether, POE(36)POP(41) dimethyl ether, POE(9)POB(2) dimethyl ether, POE(14)POB(7) dimethyl ether, POE(10)POP(10) diethyl ether, POE(10)POP(10) dipropyl ether, and POE(10)POP(10) dibutyl ether.

## Other components

In the W/O emulsion cosmetics of the present invention, other components that are normally used in cosmetics can be appropriately blended so far as the effect of the present invention is not undermined.

Examples thereof include surfactants, alcohols, moisturizers, UV absorbers, thickeners, sequestrants, various water-soluble polymers, pH adjusters, antioxidants, preservatives, perfumes, and agents such as anti-inflammatories, vitamins, amino acids, animal and plant extracts, and skin nutrients/activators.

**Product form**

The product form of the W/O emulsion cosmetics of the present invention is not limited in particular. They can be applied to any product in which W/O emulsion cosmetics containing powder are normally adopted. Preferable examples thereof include makeup cosmetics such as foundation, pre-makeup, blusher, eyeliner, and eyebrows; and sunscreen cosmetics.
In particular, from the standpoint of the feeling in use, they can be applied to liquid-type cosmetics with a relatively low viscosity, preferably 2,000 to 10,000 mPa·s (at 20 °C), and more preferably 2,000 to 6,000 mPa·s (at 20 °C).

## EXAMPLES

Hereinafter, the present invention will be further explained with reference to specific examples. However, the present invention is not limited by these examples. Modified silicones used in the test examples described below are as follows. POE represents polyoxyethylene, and POP represents polyoxypropylene. The blending quantity is expressed in mass % unless otherwise specified.

(A) Polyoxyalkylene/alkyl-comodified silicone

Modified silicone A1: Branched-chain dimethylpolysiloxane modified with lauryl groups and POE groups (KF-6038, manufactured by Shin-Etsu Chemical Co., Ltd.)

Modified silicone A2: Linear dimethylpolysiloxane modified with cetyl groups and POE-POP groups (ABIL EM 90, manufactured by Goldschmidt, Germany)

(B) Polyoxyalkylene-modified silicone

Modified silicone B1: Linear dimethylpolysiloxane modified with POE groups (KF-6017, manufactured by Shin-Etsu Chemical Co., Ltd.)

Modified silicone B2: Branched-chain dimethylpolysiloxane modified with POE groups (KF-6028, manufactured by Shin-Etsu Chemical Co., Ltd.)

The powder composition used in each test example is as follows.

| | |
|---|---|
| Fine-particle titanium oxide | 5 % by mass |
| Titanium oxide | 8 % by mass |
| Yellow iron oxide | 2 % by mass |
| Red iron oxide | 0.9 % by mass |
| Black iron oxide | 0.1 % by mass |
| Polymethylsilsesquioxane | 5 % by mass |
| TOTAL | 21 % by mass |

**Test series 1: Feeling in use**

As shown in Table 1, samples of W/O emulsion cosmetics were prepared with the use of modified silicones. The test method for the feeling in use was as follows.

(Feeling in use)

A sample immediately after preparation was applied on the skin of 20 panelists. The feeling in use was evaluated, based on the following criteria, by the number of panelists who assessed it to be "excellent".

O : 14 panelists or more
Δ : 8 to 13 panelists
X : 7 panelists or less

**Table 1**

| Component | Sample | | | | | | | |
|---|---|---|---|---|---|---|---|---|
| | 1-a# | 1-b# | 1-c# | 1-d# | 1-1# | 1-2# | 1-3# | 1-4# |
| Decamethylcyclopentasiloxane | 30 | 30 | 30 | 30 | 30 | 30 | 30 | 30 |
| Modified silicone A1 | 3 | - | - | - | 3 | - | - | - |
| Modified silicone A2 | - | 3 | - | - | - | 3 | - | - |
| Modified silicone B1 | - | - | 3 | - | - | - | 3 | - |
| Modified silicone B2 | - | - | - | 3 | - | - | - | 3 |
| Powder | - | - | - | - | 21 | 21 | 21 | 21 |
| Ethanol | 5 | 5 | 5 | 5 | 5 | 5 | 5 | 5 |
| Glycerin | 5 | 5 | 5 | 5 | 5 | 5 | 5 | 5 |
| Phenoxyethanol | 0.5 | 0.5 | 0.5 | 0.5 | 0.5 | 0.5 | 0.5 | 0.5 |
| Water | to 100 | to 100 | to 100 | to 100 | to 100 | to 100 | to 100 | to 100 |
| Feeling in use | Δ | Δ | Δ | Δ | ○ | ○ | × | × |
| # (not according to the invention) | | | | | | | | |

**Table 2**

| Component | Sample | | | | | |
|---|---|---|---|---|---|---|
| | 1-5# | 1-6# | 1-7# | 1-8# | 1-9# | 1-10# |
| Decamethylcyclopentasiloxane | 15 | 15 | 15 | 15 | 30 | 30 |
| Dimethylpolysiloxane (1mm$^2$/s, 25°C) | 15 | - | 15 | - | - | - |
| Isododecane | - | 15 | - | 15 | - | - |
| Modified silicone A1 | 3 | 3 | - | - | 0.5 | - |
| Modified silicone A2 | - | - | - | - | - | 5 |
| Modified silicone B1 | - | - | 3 | 3 | - | - |
| Modified silicone B2 | - | - | - | - | - | - |
| Powder | 21 | 21 | 21 | 21 | 21 | 21 |
| Ethanol | 5 | 5 | 5 | 5 | 5 | 5 |
| Glycerin | 5 | 5 | 5 | 5 | 5 | 5 |
| Phenoxyethanol | 0.5 | 0.5 | 0.5 | 0.5 | 0.5 | 0.5 |
| Water | to 100 | to 100 | to 100 | to 100 | to 100 | to 100 |
| Feeling in use | ○ | ○ | × | × | ○ | ○ |
| # (not according to the invention) | | | | | | |

(Production method)

The oil components (decamethylcyclopentasiloxane, dimethylpolysiloxane, and isododecane) and the modified

silicone are mixed with a homomixer (at room temperature, 9000 rpm), and the powder is added and mixed to the mixture (at room temperature, 9000 rpm), to obtain a dispersion. Separately, the aqueous components (ethanol, glycerin, phenoxyethanol, and water) are mixed and dissolved. This is added to the above-described dispersion and emulsified with a homomixer (at room temperature, 9000 rpm), and then degassed, thereby obtaining a W/O emulsion cosmetic.

As seen from Table 1, when no powder was blended, the difference in the feeling in use, depending on the types of modified silicones, was hardly present (Samples 1-a to 1-d).

On the other hand, when the powder was blended, the feeling in use varied widely depending on the types of modified silicones. Only the sample using (A) a polyoxyalkylene/alkyl-comodified silicone could easily adapt to the skin with a swift finish, and exhibited an excellent feeling in use with the right fit (Samples 1-1 and 1-2). The sample using (B) a polyoxyalkylene-modified silicone kept spreading on the skin during application with a slow finish, and the feeling of the right fit was not satisfactory (Samples 1-3 and 1-4).

As shown in Table 2, even when decamethylcyclopentasiloxane was partially replaced with other volatile oils in the system containing the powder, the excellent feeling in use was achieved only in the case that (A) a polyoxyalkylene/alkyl-comodified silicone was used (Test Examples 1-5 and 1-6). The excellent feeling in use became prominent when the blending quantity of (A) a polyoxyalkylene/alkyl-comodified silicone in the W/O emulsion cosmetics was 0.1 mass % or higher, and more prominent when the blending quantity was 0.5 mass % or higher. However, even when the excess amount was blended beyond 5 mass %, a further improvement of the effect was hardly observed.

Thus, it was considered that some sort of interaction was present between the powder and the polyoxyalkylene/alkyl-comodified silicone, affecting the feeling in use.

Therefore, the present inventors have carried out various measurements for each sample, and have found that there is a prominent difference in friction behavior.

Fig. 1 shows a comparison of kinetic friction force curves (kinetic friction force-friction time) of Samples 1-1 to 1-4. In the kinetic friction force curves of Sample 1-1 and Sample 1-2, which were excellent in the feeling in use, the kinetic friction force sharply increased from the start of friction and then maintained a nearly constant value with almost no decrease. On the other hand, in the kinetic friction force curves of Sample 1-3 and Sample 1-4, which were poor in the feeling in use, the kinetic friction force sharply increased from the start of friction, and then sharply decreased, and a distinct peak of the kinetic friction force was observed.

Fig. 2 shows a comparison of kinetic friction force curves of Sample 1-3, Sample 1-7, and Sample 1-8. Although the position of the kinetic friction force peak shifted depending upon volatile oils, there was no change in the overall patterns of kinetic friction force curves.

Thus, it was clarified that the W/O emulsion cosmetics containing powder and the polyoxyalkylene/alkyl-comodified silicone and achieving an excellent feeling in use display a characteristic pattern in the kinetic friction force curve, which shows the change of the kinetic friction force with the elapse of friction time. That is, the kinetic friction force sharply increases from the start of friction and then maintains a nearly constant value with almost no decrease. Here, the point where the slope s of the tangent line to the kinetic friction force curve becomes $0 \leqq s \leqq 0.01$ (mN/second), for the first time after the start of friction, is defined to be X, the necessary time from the start of friction to point X is defined to be Tx, and the kinetic friction force at point X is defined to be Px. Then, in such a characteristic pattern of the kinetic friction force curve, Tx is 350 seconds or less, and the maximum change of the kinetic friction force $\Delta P$ (absolute value) from Px is within 10% of Px in the range between Tx to 800 seconds. Hereinafter, such a pattern of the kinetic friction force curve is called "pattern A". The feeling in use has a trend to improve with a decrease in Tx that is the time to reach point X, and the preferable Tx is 200 seconds or less.

When (B) a polyoxyalkylene-modified silicone is used, it is possible to allow $Tx \leqq 350$ seconds. However, immediately after Tx, the kinetic friction force significantly decreases from Px. Therefore, the maximum change of the kinetic friction force $\Delta P$ drastically exceeds 10% (hereinafter, such a pattern of the kinetic friction force curve is called "pattern B"). On the other hand, when (A) a polyoxyalkylene/alkyl-comodified silicone is used, the kinetic friction force hardly decreases after Tx, and the maximum change of the kinetic friction force $\Delta P$ is very small. Thus, the modified silicones greatly affect $\Delta P$; on the other hand, Tx is hardly affected.

In Fig. 3, the point X, the Tx that is the time to reach point X, the kinetic friction force Px, and the $\Delta P$ that is the maximum change of the kinetic friction force with respect to Px are schematically shown with reference to the representative examples of pattern A and pattern B. In the present invention, the kinetic friction force curves were obtained by measuring the kinetic friction force, with a friction force measurement apparatus (manufactured by Shinto Scientific Co., Ltd.), in the repeated back-and-forth friction at about 20 °C.

**Test series 2: Emulsion stability**

In addition, the emulsion stability was tested for the samples prepared in above-described Test Example 1. The test method was as follows.

(Emulsion stability)

After the sample was allowed to stand at 20 °C for 4 weeks, the appearance was observed by the naked eye and evaluated by the following criteria.

O : No appearance change of emulsion particles such as separation or aggregation is observed.

Δ : Slight appearance change of emulsion particles such as separation or aggregation is observed.

X: Appearance change of emulsion particles such as separation or aggregation is observed.

**Table 3**

| Component | Sample | | | | | | | | | |
|---|---|---|---|---|---|---|---|---|---|---|
| | 1-1# | 1-2# | 1-3# | 1-4# | 1-5# | 1-6# | 1-7# | 1-8# | 1-9# | 1-10# |
| Decamethylcyclopentasiloxane | 30 | 30 | 30 | 30 | 15 | 15 | 15 | 15 | 30 | 30 |
| Dimethylpolysiloxane (1mm$^2$/s, 25°C) | - | - | - | - | 15 | - | 15 | - | - | - |
| Isododecane | - | - | - | - | - | 15 | - | 15 | - | - |
| Modified silicone A1 | 3 | - | - | - | 3 | 3 | - | - | 0.1 | - |
| Modified silicone A2 | - | 3 | - | - | - | - | - | - | - | 5 |
| Modified silicone B1 | - | - | 3 | - | - | - | 3 | 3 | - | - |
| Modified silicone B2 | - | - | - | 3 | - | - | - | - | - | - |
| Powder | 21 | 21 | 21 | 21 | 21 | 21 | 21 | 21 | 21 | 21 |
| Ethanol | 5 | 5 | 5 | 5 | 5 | 5 | 5 | 5 | 5 | 5 |
| Glycerin | 5 | 5 | 5 | 5 | 5 | 5 | 5 | 5 | 5 | 5 |
| Phenoxyethanol | 0.5 | 0.5 | 0.5 | 0.5 | 0.5 | 0.5 | 0.5 | 0.5 | 0.5 | 0.5 |
| Water | to 100 | to 100 | to 100 | to 100 | to 100 | to 100 | to 100 | to 100 | to 100 | to 100 |
| Emulsion stability (20°C, 4W) | × | × | ○ | Δ | × | × | ○ | Δ | × | × |
| # (not according to the invention) | | | | | | | | | | |

As shown in Table 3, when (A) a polyoxyalkylene/alkyl-comodified silicone was used, the feeling in use was excellent as in Test Example 1; however, the emulsion stability was very low.

On the other hand, when (B) a polyoxyalkylene-modified silicone was used, the feeling in use was poor; however, the emulsion stability was relatively good.

Therefore, the present inventors have further investigated the use of both in combination.

**Table 4**

| Component | Sample | | | | | | | |
|---|---|---|---|---|---|---|---|---|
| | 2-1# | 2-2# | 2-3# | 2-4# | 2-5# | 2-6# | 2-7# | 2-8# |
| Decamethylcyclopentasiloxane | 15 | 15 | 15 | 15 | 15 | 15 | 15 | 15 |
| Dimethylpolysiloxane (1mm$^2$/s, 25°C) | 15 | 15 | 15 | 15 | 15 | 15 | 15 | 15 |
| Modified silicone A1 | 1.5 | 1.5 | 1.5 | 1.5 | 1.5 | 1.5 | - | 1.5 |
| Modified silicone A2 | - | - | - | - | - | - | 1.5 | - |
| Modified silicone B1 | 0 | 0.3 | 0.8 | 3 | 6.0 | 1.5 | - | - |
| Modified silicone B2 | - | - | - | - | - | - | 1.5 | 1.5 |

(continued)

| Component | Sample | | | | | | | |
|---|---|---|---|---|---|---|---|---|
| | 2-1# | 2-2# | 2-3# | 2-4# | 2-5# | 2-6# 2-7# | | 2-8# |
| Powder | 21 | 21 | 21 | 21 | 21 | 21 | 21 | 21 |
| Methanol | 5 | 5 | 5 | 5 | 5 | 5 | 5 | 5 |
| Glycerin | 5 | 5 | 5 | 5 | 5 | 5 | 5 | 5 |
| Phenoxyethanol | 0.5 | 0.5 | 0.5 | 0.5 | 0.5 | 0.5 | 0.5 | 0.5 |
| Water | to 100 | to 100 | to 100 | to 100 | to 100 | to 100 | to 100 | to 100 |
| (B)/(A) | 0 | 0.2 | 0.5 | 2 | 4 | 1 | 1 | 1 |
| Feeling in use | ○ | ○ | ○ | ○ | Δ | ○ | ○ | ○ |
| Tx ≦ 350 secs | ○ | ○ | ○ | ○ | ○ | ○ | ○ | ○ |
| ΔP ≦ 10% | ○ | ○ | ○ | ○ | × | ○ | ○ | ○ |
| Emulsion stability (20°C, 4W) | × | Δ | ○ | ○ | ○ | ○ | Δ | Δ |

# (not according to the invention)
* With respect to each of the conditions of Tx ≤ 350 seconds and ΔP ≤ 10 %, "O" means the sample is satisfying the condition and "X" means the sample is not satisfying the condition, which are the same in the followings.

As shown in Table 4, the use of (B) a polyoxyalkylene-modified silicone in combination with (A) a polyoxyalkylene/alkyl-comodified silicone could maintain the characteristic pattern A for the kinetic friction force curve and improve emulsion stability without detriment to the excellent feeling in use. However, when an excess amount of (B) a polyoxyalkylene-modified silicone was blended with respect to (A) a polyoxyalkylene/alkyl-comodified silicone, the kinetic friction force curve was turned into the pattern B, and the feeling in use was undermined.

Thus, it is preferable to blend (B) a polyoxyalkylene-modified silicone in the range of 0.2 to 3 times in mass with respect to (A) a polyoxyalkylene/alkyl-comodified silicone, and it is more preferable to blend 0.5 to 2 times in mass.

**Test series 3: Volatile component**

In addition, the effect of volatile components such as ethanol and a volatile oil was investigated.

As a result, as shown in Table 5, the maximum change ΔP of the kinetic friction force was hardly affected by the change in the blending quantity of ethanol or a volatile oil. However, if the blending quantity was small, Tx exceeded 350 seconds and in such a case also, the feeling in use became lower by the feeling-in-use test by the panelists. Accordingly, the volatile components affect Tx, and both Tx and ΔP are considered to contribute to the feeling in use characteristic to the present invention. On the other hand, when the amount of ethanol or volatile oil was large, the emulsion stability decreased.

Thus, the amount of ethanol in the W/O emulsion cosmetics is preferably 1 to 20 mass %, more preferably 2 to 10 mass %, and most preferably 3 to 8 mass %. The amount of a volatile oil in the W/O emulsion cosmetic is preferably 10 to 50 mass %, more preferably 20 to 40 mass %, and most preferably 25 to 35 mass %.

**Table 5**

| Component | Sample | | | | | |
|---|---|---|---|---|---|---|
| | 3-1# | 3-2# | 3-3# | 3-4# | 3-5# | 3-6# |
| Decamethylcyclopentasiloxane | 15 | 15 | 15 | 3 | 10 | 25 |
| Dimethylpolysiloxane (1mm$^2$/s, 25°C) | 15 | 15 | 15 | 3 | 10 | 25 |
| Modified silicone A1 | 1.5 | 1.5 | 1.5 | 1.5 | 1.5 | 1.5 |
| Modified silicone B 1 | 1.5 | 1.5 | 1.5 | 1.5 | 1.5 | 1.5 |
| Powder | 21 | 21 | 21 | 21 | 21 | 21 |
| Ethanol | 0 | 1 | 20 | 5 | 5 | 5 |

(continued)

| Component | Sample | | | | | |
|---|---|---|---|---|---|---|
| | 3-1# | 3-2# | 3-3# | 3-4# | 3-5# | 3-6# |
| Glycerin | 5 | 5 | 5 | 5 | 5 | 5 |
| Phenoxyethanol | 0.5 | 0.5 | 0.5 | 0.5 | 0.5 | 0.5 |
| Water | to 100 | to 100 | to 100 | to 100 | to 100 | to 100 |
| Feeling in use | × | ○ | ○ | × | ○ | o |
| ΔP ≦ 10% | ○ | ○ | ○ | ○ | ○ | ○ |
| Tx ≦ 350 secs | × | ○ | ○ | × | ○ | ○ |
| Emulsion stability (20°C, 4W) | ○ | ○ | Δ | ○ | ○ | Δ |
| # (not according to the invention) | | | | | | |

**Test series 4: Sodium glutamate**

As described above, when (B) a polyoxyalkylene-modified silicone was used in combination with (A) a polyoxyalkylene/alkyl-comodified silicone in a specific ratio, W/O emulsion cosmetics with good emulsion stability could be obtained without detriment to the excellent feeling in use. Therefore, the dispersion stability of powder and viscosity stability of compositions were further investigated. The test methods are as follows.

(Dispersion stability of powder)

About 30 mL of a sample was placed in a 50 mL screw vial (diameter: 3 cm) and closed, and the rolling test was carried out by rotating at a speed of 45 rpm for 4 hours at 20 °C. Then, the dispersibility of powder was evaluated, by naked-eye observation, based on the following criteria.

○ : No color bands (yellow lines and red unevenness) are observed.
Δ : Slight color bands (yellow lines and red unevenness) are observed.
X: Color bands (yellow lines and red unevenness) are observed.

(Viscosity stability)

The sample viscosity was measured immediately after preparation and after the stationary storage at 50 °C for 4 weeks. The decreased rate of the viscosity after the stationary storage ($V_t$) with respect to the viscosity immediately after preparation ($V_0$) was calculated by the following equation, and the viscosity stability was evaluated by the following criteria. The viscosity was measured at 25 °C with a single cylindrical rotational viscometer, which was manufactured by Shibaura Systems Co., Ltd.

$$\text{Decreased rate of viscosity (\%)} = [(V_0 - V_t)/V_0] \times 100$$

○ : The decreased rate of viscosity is lower than 15%.
Δ : The decreased rate of viscosity is 15% or higher and lower than 30%.
X : The decreased rate of viscosity is 30% or higher and lower than 60%.
XX : The decreased rate of viscosity is 60% or higher.

**Table 6**

| Component | Sample | | | | | | |
|---|---|---|---|---|---|---|---|
| | 4-1# | 4-2# | 4-3# | 4-4# | 4-5# | 4-6# | 4-7# |
| Decamethylcyclopentasiloxane | 12.25 | 12.25 | 12.25 | 12.25 | 12.25 | 12.25 | 12.25 |
| Dimethylpolysiloxane (1mm²/s, 25°C) | 12.25 | 12.25 | 12.25 | 12.25 | I 12.25 | 12.25 | 12.25 |
| Modified silicone A1 | 1.5 | 1.5 | 1.5 | 1.5 | 1.5 | 1.5 | 1.5 |
| Modified silicone B1 | 1.5 | 1.5 | 1.5 | 1.5 | 1.5 | 1.5 | 1.5 |
| Powder | 21 | 21 | 21 | 21 | 21 | 21 | 21 |
| Ethanol | 5 | 5 | 5 | 5 | 5 | 5 | 5 |
| Glycerin | 5 | 5 | 5 | 5 | 5 | 5 | 5 |
| Phenoxyethanol | 0.5 | 0.5 | 0.5 | 0.5 | 0.5 | 0.5 | 0.5 |
| Sodium glutamate | - | 1 | - | - | - | - | - |
| EDTA-3Na | - | - | 1 | - | - | - | - |
| NaCl | - | - | - | 1 | - | - | - |
| KCl | - | - | - | - | 1 | - | - |
| $MgSO_4$ | - | - | - | - | - | 1 | - |
| $CaCl_2$ | - | - | - | - | - | - | 1 |
| Water | to 100 | to 100 | to 100 | to 100 | to 100 | to 100 | to 100 |
| Dispersion stability of powder | × (Yellow lines) | ○ | × (Yellow lines) | Δ (Yellow lines) | ○ | × (Red unevenness) | × (Red uneverness) |
| Viscosity stability | ×× | ○ | ○ | Δ | × | Δ | × |
| # (not according to the invention) | | | | | | | |

As shown in Table 6, the color bands, which is due to powder aggregation, or a decrease in viscosity were sometimes observed. However, there were cases in that the color bands due to powder aggregation were suppressed or the viscosity stability was improved, by blending a salt. In particular, sodium glutamate showed good effects to both properties.

According to the investigation of the present inventors, if the amount of sodium glutamate is too small, the effect was not satisfactory. When the amount was too large, a significant increase in the effect could not be obtained. Therefore, the amount of sodium glutamate in the W/O type emulsion cosmetic is preferably 0.1 to 5 mass %, more preferably 0.2 to 3 mass %, and most preferably 0.5 to 2 mass %. If the amount of sodium glutamate was within the range, sodium glutamate did not affect the friction behavior, which is characteristic to the W/O emulsion cosmetics of the present invention.

**Test series 5: Organically modified clay mineral**

The blending of an organically modified clay mineral was also investigated.

Organically modified hectorite [Bentone 38VCG (Elementis Specialties, Inc. (UK))] was added to decamethylcyclopentasiloxane so that the concentration was 10 mass %, and the mixture was treated with a bead mill by using the same volume of glass beads with a diameter of 1 mm as the media, to obtain a dispersion of organically modified hectorite with the average thickness 0.1 μm or less and the average length of 0.5 to 50 μm (organically modified hectorite fine dispersion), which was used as the organically modified clay mineral.

**Table 7**

| Component | Sample | | | | | | |
|---|---|---|---|---|---|---|---|
| | 5-1# | 5-2# | 5-3# | 5-4# | 5-5# | 5-6 | 5-7# |
| Decamethylcyclopentasiloxane | 12.25 | 3.25 | 3.25 | 3.25 | 3.25 | 3.25 | 3.25 |
| Dimethylpolysiloxane (1mm$^2$/s, 25°C) | 12.25 | 12.25 | 12.25 | 12.25 | 12.25 | 12.25 | 12.25 |
| Modified silicone A1 | 1.5 | 1.5 | 1.5 | 1.5 | 1.5 | 1.5 | 1.5 |
| Modified silicone B1 | 1.5 | 1.5 | 1.5 | 1.5 | 1.5 | 1.5 | 1.5 |
| Powder | 21 | 21 | 21 | 21 | 21 | 21 | 21 |
| Ethanol | 5 | 5 | 5 | 5 | 5 | 5 | 5 |
| Glycerin | 5 | 5 | 5 | 5 | 5 | 5 | 5 |
| Phenoxyethanol | 0.5 | 0.5 | 0.5 | 0.5 | 0.5 | 0.5 | 0.5 |
| Organically modified hectorite fine dispersion (Net organically modified hectorite) | - (-) | 10 (1) | 10 (1) | 10 (1) | 10 (1) | 10 (1) | 10 (1) |
| Isostearic acid | - | - | 1 | - | - | - | - |
| Sorbitan sesquiisostearate | - | - | - | 1 | - | - | - |
| Bis-butyldimethicone polyglyceryl-3 | - | - | | - | 1 | - | - |
| Diglyceryl diisostearate | - | - | - | - | - | 1 | - |
| Glyceryl monoisostearate | - | - | - | - | - | - | 1 |
| Water | to 100 | to 100 | to 100 | to 100 | to 100 | to 100 | to 100 |
| Feeling in use | ○ | × | × | × | × | ○ | × |
| Powder dispersion stability | × | ○ | ○ | ○ | ○ | ○ | ○ |
| Viscosity stability | ×× | ○ | ○ | ○ | ○ | ○ | ○ |
| Viscosity immediately after preparation (mPa.s)* | 4090 | 4020 | 4200 | 5180 | 5200 | 5210 | 7010 |
| # (not according to the invention)<br>*at 25°C, measured by a single cylindrical rotational viscometer (manufactured by Shibaura Systems Co., Ltd.) | | | | | | | |

(Production method)

The oil components (decamethylcyclopentasiloxane, dimethylpolysiloxane, higher fatty acids, and ester oils) and the modified silicones are mixed with a homomixer (at room temperature, 9000 rpm). To this mixture, the organically modified hectorite fine dispersion is added and mixed (at room temperature, 9000 rpm), and then the powder is added and mixed to the mixture to obtain a dispersion (at room temperature, 9000 rpm). Separately, the aqueous components (ethanol, glycerin, phenoxyethanol, and water) are mixed and dissolved. This is added to the above-described dispersion, emulsified with a homomixer (at room temperature, 9000 rpm), and then degassed to obtain a W/O emulsion cosmetic.
As shown in Table 7, it was possible to improve the powder dispersion stability and viscosity stability by blending an organically modified hectorite. In order to obtain such an effect, 0.05 mass % or more of organically modified hectorite are blended into the cosmetic, and it is preferable to blend 0.1 mass % or more.
The feeling in use had a trend to deteriorate with an increase in the blending quantity of the organically modified hectorite.
As a result of the investigation by the present inventors, it was clarified that if diglyceryl diisostearate is used in combination with an organically modified hectorite, the deterioration in the feeling in use can be suppressed without detriment to the improving effect on the dispersion stability and viscosity stability by the blending of an organically modified hectorite.
If the amount of diglyceryl diisostearate was too small, the effect could not be satisfactorily achieved. If the amount was too large, the corresponding improvement in the effect could not be obtained. Accordingly, the amount of diglyceryl diisostearate is 1 to 10 times in mass with respect to the organically modified hectorite.

**Test series 6: Moisturizing property**

W/O liquid foundations having the compositions in Table 8 were prepared, and the moisturizing effect was tested. As a result, although Sample 6-1 contains the moisturizer glycerin, the moisturizing effect was hardly observed. Therefore, the improvement of the moisturizing property was investigated.

**Table 8**

| Component | Sample | |
|---|---|---|
| | 6-1 | 6-2 |
| Decamethylcyclopentasiloxane | 5.5 | 5.5 |
| Dimethylpolysiloxane (1mm$^2$/s, 25°C) | 10 | 10 |
| Modified silicone A1 | 1.5 | 1.5 |
| Modified silicone B1 | 1.5 | 1.5 |
| Organically modified hectorite fine dispersion * | 2 | 2 |
| (Net organically modified hectorite) | (0.2) | (0.2) |
| Diisostearyl diisostearate | 1 | 1 |
| Powder | 21 | 21 |
| Glycerin | 5 | - |
| Ethanol | 5 | 5 |
| Sodium glutamate | 1 | 1 |
| Phenoxyethanol | 0.5 | 0.5 |
| Water | to 100 | to 100 |
| Moisturizing effect | × | × |

(Moisturizing effect test)

A sample (2 μg/cm$^2$) was applied on the medial side of the upper arm. Immediately before the application and 4 hours after the application, the water content of the stratum corneum was measured with a skin moisture meter (Corneometer: manufactured by Courage + Khazaka Electronic GmbH, Germany) (N= 4).

The moisturizing effect of each sample was evaluated from the ratio, (average water content of the stratum corneum 4 hours after the application)/(average water content of the stratum corneum immediately before the application), based on the following criteria.

○ : 1.2 or more

Δ : 1.1 or more to less than 1.2

X : less than 1.1

**Table 9**

| Test component | Moisturizing effect | Feeling in use |
|---|---|---|
| <Nonvolatile oil> | | |
| Liquid paraffin | ○ | × |
| Cetyl octanoate | Δ | × |
| Di-2-ethylhexyl succinate | ○ | ○ |
| Diisopropyl sebacate | Δ | × |
| Glyceryl tri-2-ethylhexanoate | ○ | × |
| Pentaerythritol tetra-2-ethylhexanoate | Δ | × |
| Tripropylene glycol pivalate | ○ | × |
| 2-Ethylhexyl cinnamate | ○ | × |
| Methylphenylpolysiloxane (15 $mm^2$/s) | ○ | ○ |
| Dimethylpolysiloxane (6 $mm^2$/s) | ○ | ○ |
| Hydrogenated polyisobutene | × | × |
| <Volatile oil> | | |
| Decamethylcyclopentasiloxane | × | ○ |
| Dodecamethylcyclohexasiloxane | × | ○ |
| <Water-soluble moisturizer> | | |
| 1,3-Butylene glycol | × | × |

Table 9 shows the test results of the moisturizing effect and the feeling in use when 10 mass % of the test component was blended into the above-described Sample 6-1 (the increase by the blending of a test component was adjusted by decreasing the amount of water so that the total will be 100 mass %).

As shown in Table 9, when a nonvolatile oil was used in combination with glycerin, the water content of the stratum corneum 4 hours after the application increased, thereby obtaining the moisturizing effect. On the other hand, if only nonvolatile oil was blended without blending glycerin, the moisturizing effect could not be obtained. Therefore, it is considered that the moisturizing effect of glycerin is satisfactorily achieved because of the residual oil in the cosmetic film.

As seen from Table 9, however, the nonvolatile oils that can significantly increase the moisturizing effect of glycerin, while maintaining the kinetic friction force curve pattern A characteristic to the present invention and achieving satisfactorily the excellent feeling in use, were only diethylhexyl succinate, methylphenylpolysiloxane, and dimethylpolysiloxane.

Accordingly, it is preferable in the present invention that one or more nonvolatile oils selected from the group consisting of diethylhexyl succinate, methylphenylpolysiloxane, and dimethylpolysiloxane is contained in combination with glycerin.

According to the investigation of the present inventors, the effect cannot be satisfactorily achieved if the blending quantity of the above-described specific nonvolatile component is too small. Even when the blending quantity is too much, the corresponding improvement in the effect cannot be achieved. Therefore, the sum of diethylhexyl succinate,

methylphenylpolysiloxane, and dimethylpolysiloxane in the W/O emulsion cosmetics is preferably 0.1 to 10 mass %, more preferably 2 to 8 mass %, and most preferably 3 to 5 mass %.

In addition, a nonvolatile oil other than diethylhexyl succinate, methylphenylpolysiloxane, and dimethylpolysiloxane can be blended so far as the effect of the present invention is not undermined.

The blending quantity of glycerin is suitably determined according to the desired moisturizing effect. The blending quantity in the W/O emulsion cosmetic is normally 0.1 to 20 mass %, and preferably 1 to 10 mass %. If the blending quantity is too small, the moisturizing effect is small. Even when the excess amount is blended, the corresponding improvement in the effect cannot be achieved.

In addition, other water-soluble moisturizers other than glycerin can be blended so far as the effect of the present invention is not undermined. However, if a large amount of dihydric glycols such as 1,3-butylene glycol is blended, the feeling in use tends to significantly deteriorate. Therefore, when a dihydric glycol is blended, the amount thereof in the cosmetic is preferably 3 mass % or less, and more preferably 1 mass % or less.

**Formulation example 1** (not according to the invention)

**Table 10**

| Component | Amount |
|---|---|
| <Oil component> | |
| Decamethylcyclopentasiloxane | 15 |
| Dimethylpolysiloxane (1mm$^2$/s, 25°C) | 10 |
| Diethylhexyl succinate | 2 |
| Octyl methoxycinnamate | 4 |
| Dipentaerythrityl hexahydroxystearate | 1 |
| POE(36)POP(41)dimethyl ether | 0.5 |
| <Modified silicone> | |
| Polyoxyalkylene/alkyl co-modified silicone | 2 |
| Polyoxyalkylene modified silicone | 2 |
| Diglyceryl diisostearate | 0.5 |
| <Powder> | |
| Powder* | 21 |
| <Aqueous component> | |
| Ethanol | 5 |
| Glycerin | 5 |
| Xylitol | 0.5 |
| Erythritol | 0.5 |
| Sodium glutamate | 1 |
| Phenoxyethanol | 0.5 |
| Water | to 100 |
| * same with Test Example 1 | |

The oil components and the modified silicones were mixed with a homomixer (at room temperature, 9000 rpm), and the powder is added and mixed thereto (at room temperature, 9000 rpm), to obtain a dispersion. Separately, the aqueous components were mixed and dissolved. This is added to the above-described dispersion, emulsified with a homomixer (at room temperature, 9000 rpm), and degassed to obtain a W/O emulsion cosmetic.

The cosmetic of Formulation Example 1 displayed a kinetic friction curve of pattern A, easily adapted to the

skin with a swift finish, and achieved a feeling of the right fit in the feeling of use.

## Claims

1. A W/O emulsion cosmetic, comprising the following components:

(a) 0.1 to 5 mass % of polyoxyalkylene/alkyl-comodified silicone represented by the general formula (I)

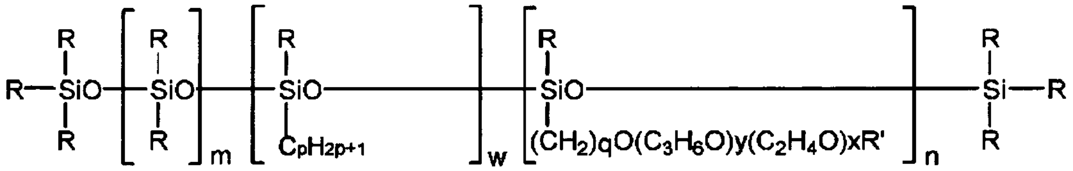

· · · (I)

in which

R is an alkyl group of 1 to 3 carbon atoms or a phenyl group;
R' is a hydrogen atom or an alkyl group of 1 to 12 carbon atoms;
p is 6 to 30;
q is 1 to 50;
m is 1 to 100;
n, w, and x are 1 to 50, respectively; and
y is 1 to 50;

(b) polyoxyalkylene-modified silicone represented by the general formula (II)

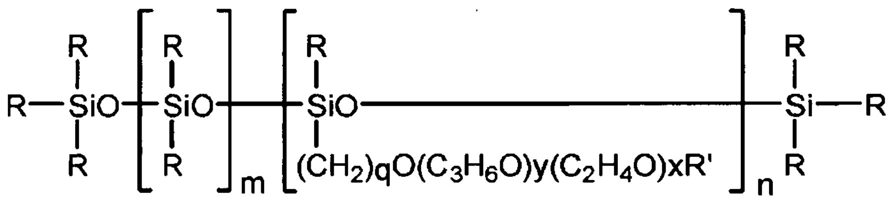

· · · (II)

in which

R is an alkyl group of 1 to 3 carbon atoms or a phenyl group;
R' is a hydrogen atom or an alkyl group of 1 to 12 carbon atoms;
q is 1 to 50;
m is 1 to 100;
n and x are 1 to 50, respectively; and
y is 1 to 50;
wherein the blending quantity of the polyoxyalkylene-modified silicone is 0.2 to 3 times in mass with respect to the blending quantity of the polyoxyalkylene/alkyl-comodified silicone;

(c) 5 to 50 mass % of powder;
(d) 10 to 50 mass % of a volatile oil having a boiling point of 260°C or lower at ordinary pressure;
(e) 1 to 20 mass % of ethanol;
(f) water,
(g) 0.05 to 1 mass % of an organically modified hectorite, and
(h) diglyceryl diisostearate, wherein the blending quantity of the diglyceryl diisostearate is 1 to 10 times in mass with respect to the blending quantity of the organically modified hectorite.

2. The W/O emulsion cosmetic of claim 1, further comprising 0.1 to 5 mass % of sodium glutamate.

3. The W/O emulsion cosmetic of claim 1 or 2, further comprising glycerin and one or more nonvolatile oils selected

from the group consisting of diethylhexyl succinate, methylphenylpolysiloxane, and dimethylpolysiloxane.

**4.** The W/O emulsion cosmetic of any of claims 1 to 3, wherein a dihydric glycol in the cosmetic is 3 mass % or less.

**Patentansprüche**

**1.** Kosmetikum in Form einer W/O-Emulsion, umfassend die nachfolgenden Komponenten:

(a) 0.1 bis 5 Masse% eines durch die allgemeine Formel (I) dargestellten Polyoxyalkylen/Alkyl-comodifizierten Silikons

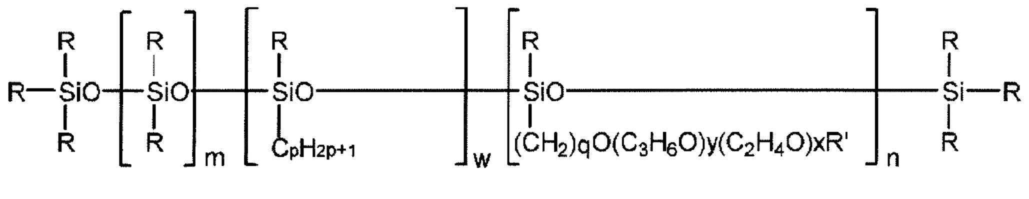

· · · (I)

in welchem

R für eine Alkylgruppe mit 1 bis 3 Kohlenstoffatomen oder eine Phenylgruppe steht;
R' für ein Wasserstoffatom oder eine Alkylgruppe mit 1 bis 12 Kohlenstoffatomen steht;
p für 6 bis 30 steht;
q für 1 bis 50 steht;
m für 1 bis 100 steht;
n, w und x jeweils für 1 bis 50 stehen; und
y für 1 bis 50 steht;

(b) ein durch die allgemeine Formel (II) dargestelltes Polyoxyalkylen-modifiziertes Silikon

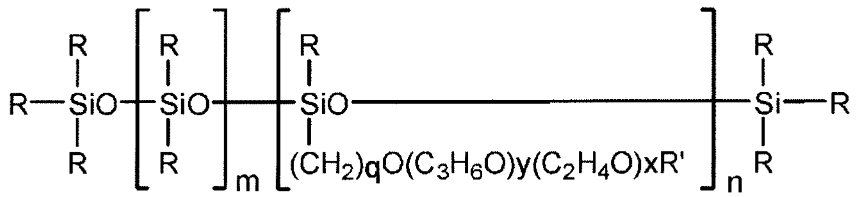

· · · (II)

in welchem

R für eine Alkylgruppe mit 1 bis 3 Kohlenstoffatomen oder eine Phenylgruppe steht;
R' für ein Wasserstoffatom oder eine Alkylgruppe mit 1 bis 12 Kohlenstoffatomen steht;
q für 1 bis 50 steht;
m für 1 bis 100 steht;
n und x jeweils für 1 bis 50 stehen; und
y für 1 bis 50 steht;
wobei die eingemischte Menge an Polyoxyalkylenmodifiziertem Silikon das 0.2- bis 3-fache, bezogen auf die Masse, der eingemischten Menge an Polyoxyalkylen/Alkyl-comodifiziertem Silikon beträgt;

(c) 5 bis 50 Masse% an Pulver;
(d) 10 bis 50 Masse% eines flüchtigen Öls mit einem Siedepunkt von 260°C oder höher bei Normaldruck;
(e) 1 bis 20 Masse% an Ethanol;
(f) Wasser,
(g) 0.05 bis 1 Masse% eines organisch modifizierten Hectorits, und
(h) Diglyceryldiisostearat, wobei die eingemischte Menge an Diglyceryldiisostearat das 1- bis 10-fache, bezogen auf die Masse, der eingemischten Menge an organisch modifiziertem Hectorit beträgt.

2. Kosmetikum in Form einer W/O-Emulsion nach Anspruch 1, weiterhin umfassend 0.1 bis 5 Masse% an Natriumglutamat.

3. Kosmetikum in Form einer W/O-Emulsion nach Anspruch 1 oder 2, weiterhin umfassend Glycerin und ein oder mehrere nicht-flüchtige Öle ausgewählt aus der Gruppe bestehend aus Diethylhexylsuccinat, Methylphenylpolysiloxan und Dimethylpolysiloxan.

4. Kosmetikum in Form einer W/O-Emulsion nach einem der Ansprüche 1 bis 3, wobei das Kosmetikum 3 Masse% oder weniger eines zweiwertigen Glykols enthält.

## Revendications

1. Produit cosmétique en émulsion E/H, comprenant les composants suivants :

(a) de 0,1 à 5 % en masse d'un silicone comodifié par un polyoxyalkylène/alkyle représenté par la formule générale (I)

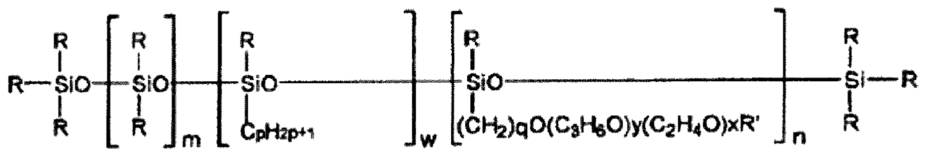
· · · (I)

dans laquelle

R est un groupe alkyle de 1 à 3 atomes de carbone ou un groupe phényle ;
R' est un atome d'hydrogène ou un groupe alkyle de 1 à 12 atomes de carbone ;
p vaut de 6 à 30 ;
q vaut de 1 à 50 ;
m vaut de 1 à 100 ;
n, w et x valent respectivement de 1 à 50 ; et
y vaut de 1 à 50 ;

(b) un silicone modifié par un polyoxyalkylène représenté par la formule générale (II)

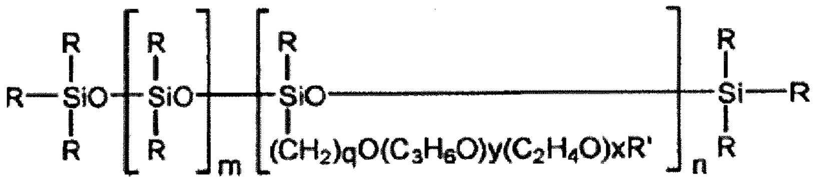
· · · (II)

dans laquelle

R est un groupe alkyle de 1 à 3 atomes de carbone ou un groupe phényle ;
R' est un atome d'hydrogène ou un groupe alkyle de 1 à 12 atomes de carbone ;
q vaut de 1 à 50 ;
m vaut de 1 à 100 ;
n et x valent respectivement de 1 à 50 ; et
y vaut de 1 à 50 ;
dans lequel la quantité de mélange du silicone modifié par un polyoxyalkylène est de 0,2 à 3 fois en masse par rapport à la quantité de mélange du silicone comodifié par un polyoxyalkylène/alkyle ;

(c) de 5 à 50 % en masse de poudre ;
(d) de 10 à 50 % en masse d'une huile volatile ayant un point d'ébullition de 260 °C ou moins à la pression ordinaire ;
(e) de 1 à 20 % en masse d'éthanol ;

(f) de l'eau,
(g) de 0,05 à 1 % en masse d'une hectorite modifiée organiquement, et
(h) du diisostéarate de diglycéryle, dans lequel la quantité de mélange du diisostéarate de diglycéryle est de 1 à 10 fois en masse par rapport à la quantité de mélange de l'hectorite modifiée organiquement.

2. Produit cosmétique en émulsion E/H selon la revendication 1, comprenant en outre de 0,1 à 5 % en masse de glutamate de sodium.

3. Produit cosmétique en émulsion E/H selon la revendication 1 ou 2, comprenant en outre de la glycérine et une ou plusieurs huiles non volatiles choisies dans le groupe constitué de succinate de diéthylhexyle, de méthylphénylpolysiloxane et de diméthylpolysiloxane.

4. Produit cosmétique en émulsion E/H selon l'une quelconque des revendications 1 à 3, dans lequel un glycol dihydrique représente 3 % en masse ou moins dans le produit cosmétique.

FIG. 1

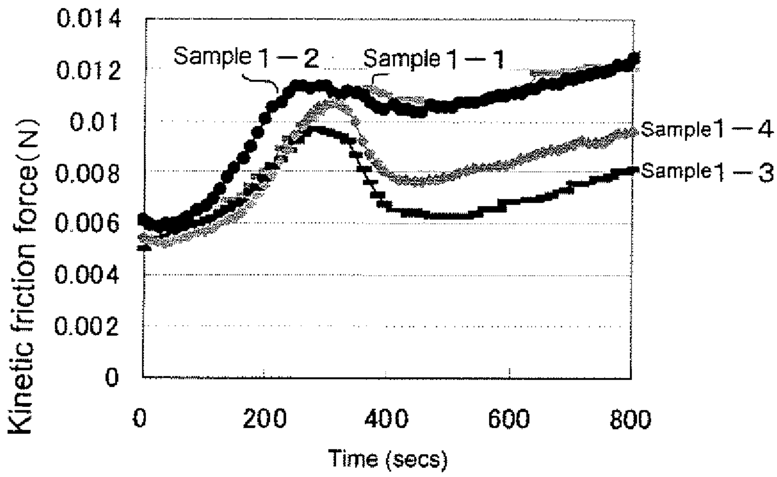
0.014
0.012
0.01
0.008
0.006
0.004
0.002
0
Sample 1 − 2
Sample 1 − 1
Sample1 − 4
Sample1 − 3
Kinetic friction force (N)
0
200
400
600
800
Time (secs)

FIG. 2

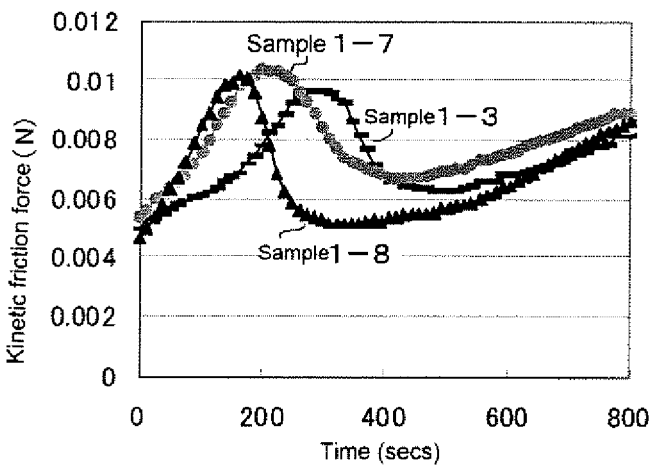
0.012
0.01
0.008
0.006
0.004
0.002
0
Sample 1 − 7
Sample1 − 3
Sample1 − 8
Kinetic friction force (N)
0
200
400
600
800
Time (secs)

FIG. 3

(a) Pattern A

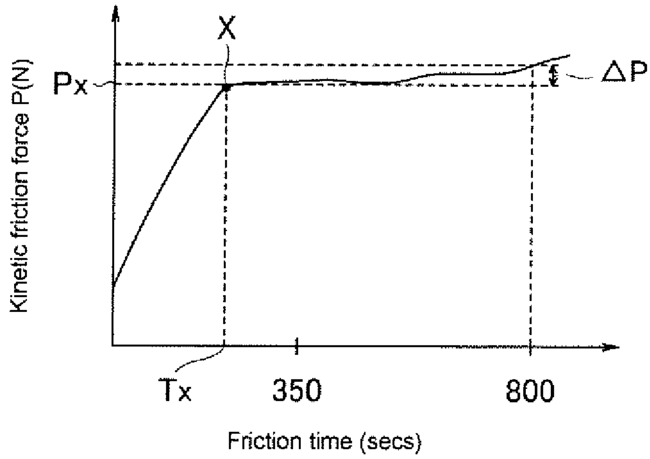


(b) Pattern B

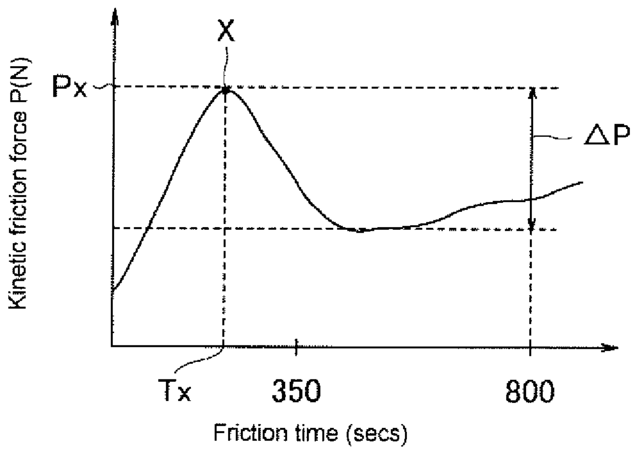

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010028496 A
- JP S63250311 A
- JP 2009040720 A
- JP 2004083541 A
- JP 2006265135 A